# FASCICULE DE BREVET EUROPEEN

(11) **EP 0 668 269 B1**
(45) Date de publication et mention de la délivrance du brevet: **29.05.2002**
(21) Numéro de dépôt: 95102219.3
(22) Date de dépôt: 17.02.1995
(51) Int. Cl.: C07D 231/44, C07D 233/88, C07D 231/40

(54) **Procédé de sulfinylation de composés hétérocycliques**
Verfahren zur Sulfinylierung von heterocyclischen Verbindungen
Process for the sulfinylation of heterocyclic compounds

(30) Priorité: 22.02.1994 FR 9402222
(43) Date de publication de la demande: 23.08.1995
(73) Titulaire: AVENTIS CROPSCIENCE S.A., 69009 Lyon (FR)
(72) Inventeur: Casado, Michel, F-69360 St-Symphorien d'Ozon (FR); Le Roy, Pierre, F-69003 Lyon (FR); Pevere, Virginie, 69008 Lyon (FR)

(56) Documents cités:
- EP-A- 0 295 117
- EP-A- 0 346 683
- WO-A-93/06089
- JOURNAL OF ORGANIC CHEMISTRY., vol.45, no.26, 19 Décembre 1980, EASTON US pages 5336 - 5339 O. CARMONA ET AL. 'Synthesis and Rearrangement of 2-(Arylsulfinyl)- and 2-(Alkylsulfinyl)pyrroles'
- JOURNAL OF ORGANIC CHEMISTRY., vol.48, no.25, 16 Décembre 1983, EASTON US pages 4803 - 4807 K.K. ANDERSEN ET AL. 'Substitution at Tricoordinate Sulfur(IV). Rearrangement of Sulfinanilides to Anilino Sulfoxides'

## Description

La présente invention concerne un nouveau procédé de sulfinylation de composés hétérocycliques. La sulfinylation des composés hétérocycliques, c'est à dire l'incorporation d'un groupe RS(O)-, est classiquement réalisée par action d'un produit de formule RSX (R et X étant définis ci-dessous) sur le composé hétérocyclique portant un atome d'hydrogène sur le carbone à substituer. Cette réaction conduit donc au composé sulfényl hétérocycle que l'on doit oxyder pour donner le composé sulfinyl désiré. Or, il se trouve que cette étape d'oxydation est souvent difficile. De plus, le composé RSX a, dans certains cas, été trouvé comme étant très toxique. C'est le cas par exemple du composé CF₃SCl qui rend les manipulations très délicates. Un autre procédé classique consiste à passer par un composé disulfure intermédiaire, coupé au niveau de la liaison S-S par un composé RX ce qui donne le composé sulfényl, lequel est ensuite oxydé en composé sulfinyl. Ce procédé évite le recours au composé RSX mais n'évite pas l'étape d'oxydation ultérieure.

### But de l'invention :

C'est donc l'objet de la présente invention que d'éviter les deux inconvénients ci-dessus (oxydation difficile et toxicité du réactif) en proposant un procédé de sulfinylation directe par action de RS(O)X sur des composés hétérocycliques sans nuire au rendement global de la réaction et tout en réduisant le nombre d'étapes réactionnelles. Il a maintenant été trouvé, et c'est ce qui constitue l'objet de la présente invention, un procédé qui répond totalement ou en partie à la satisfaction de ces objectifs de facilité, de rentabilité et de sécurité.

### Contexte technique et art antérieur:

Les demandes de brevets européens EP 0295117, 0460940, 0484165 donnent de nombreux exemples d'accès aux composés hétérocycliques sulfinylés. Deux types de procédés apparaissent alors:

Un premier groupe de procédé consiste à produire le composé sulfénylé que l'on doit oxyder par la suite pour obtenir le composé désiré. La préparation des composés sulfénylés se fait soit par action direct d'un réactif RSX sur le composé hétérocyclique ayant un atome d'hydrogène en position à sulfényler, soit par action d'organomagnésiens ou de composés R'I, R'Br (R' étant un groupe alkyl ou haloalkyl) sur un thiocyanatohétérocycle, soit enfin par réduction de composés disulfures en présence de composés R'I. Ces divers procédés sont décrits dans la demande européenne EP A 0295117 (cf. procédé b, d1, d2 et d3 en pages 11-12). Un autre procédé utilisant des composés disulfures est décrit dans le brevet européen EP B1 374061.

Un deuxième groupe consiste à faire réagir un composé sulfinylé sur un composé particulier de telle sorte que le produit de cette réaction formera par cyclisation le composé hétérocyclique sulfinylé. Pour le détail de ces procédés, on se reportera à nouveau à la demande européenne EP A 0295117 (cf. procédé a et c en page 11).

Par ailleurs, on connait, en série aromatique et non hétérocyclique, des références faisant état de sulfinylation directe d'aromatiques (cycle phényl). Par exemple, dans Bull. Chem. Soc. Jpn, 46, (1973) 3615, il est décrit la réaction de sulfinylation du methoxybenzène par du chlorure de p-méthylphénylsulfinyl en présence de catalyseur AlCl₃. Une autre réaction de ce type est décrite dans Zh Org Khim, 17 (9) (1981) 1800. La réaction met ici en présence un réactif de Grignard qui est le bromure de paraméthylbenzène magnésien sur du chlorure de naphtylsulfinyl.

Des réactions d'akylsulfinylation directe d'hétéroaromatiques, donnant des rendements faibles de l'ordre de 20%, sont connues de la demande européenne EP A 0 346 683 et du journal J. Org. Chem. 45 (1980) 5336. Des réactions dans lesquelles un dérivé phénylsulfinamide se réarrange pour conduire à un dérivé phényl ayant un groupe amino porté par un atome de carbone et un groupe sulfinyl sur le carbone vicinal sont connues du journal J. Org. Chem. 48 (1983) 4803.

### Description de la présente invention :

L'invention concerne un nouveau procédé de sulfinylation de composés hétérocycliques qui consiste à faire réagir :
- un dérivé de formule RS(O)X, dans laquelle R est un groupe halométhyle, X est un atome d'halogène, le groupe hydroxy ou l'un de ses sels, un groupe dialkylamino NR₂R₃, R₂ et R₃ étant des groupes alkyl ou haloalkyl de 1 à 4 atomes de carbone, ou un groupe aryloxy, dans lequel la partie aryl correspond de préférence à un groupe phényl, éventuellement substitué par un ou plusieurs atomes d'halogène ou groupes alkyl ou haloalkyl de 1 à 4 atomes de carbone,
- avec un composé hétérocyclique Het choisi dans le groupe comprenant les pyrroles, les pyrazoles, les imidazoles, les oxazoles, les isoxazoles, les isothiazoles, les thiazoles, les triazoles, tous ces hétérocycles Het étant éventuellement substitués par un ou plusieurs atomes ou groupes choisi parmi halogène, amine, mono- ou di-alkylamino, nitrile, aryl, aryl substitués par un ou plusieurs atome d'halogène et/ou un ou plusieurs groupes alkyl, haloalkyl ou SF₅.

Un procédé préféré consiste à choisir l'hétérocycle Het dans le groupe comprenant les pyrroles, les pyrazoles, les imidazoles et dans ce cas un composé C, choisi dans le groupe comprenant les tosylates, les chlorhydrates, les mésylates de diméthylamine, de pyridine, de triméthylamine, de diéthylamine, d'isopropylamine ou de toute autre amine primaire, secondaire ou tertiaire, ou l'acide chlorhydrique gazeux, éventuellement en présence d'une quantité environ équimolaire d'acide paratoluènesulfonique, peut être ajouté pour parfaire la réaction.

Un réactif choisi dans le groupe comprenant le phosgène COCl₂, les chloroformiates, le PCl₅ ou le SOCl₂ peut éventuellement être utilisé pour faire les réactions précitées.

D'une manière avantageuse, on choisit Het = amino pyrazole. Ce groupe réagit alors avec RS(O)X pour donner un composé sulfinamide se réarrangeant par la suite pour conduire à un composé hétérocyclique ayant un groupe amino porté par un atome de carbone et un groupe sulfinyl RS(O) sur le carbone vicinal.

Selon un mode de réalisation particulier de l'invention, on utilise le composé CF₃S(O)X dans lequel X est l'atome de chlore. On sait que dans ce cas le composé CF₃S(O)Cl est moins toxique que le composé CF₃SCl utilisé auparavant, ce qui constitue un des avantages de la présente invention. Outre cela, on évite également, et cela a déjà été signalé, l'étape d'oxydation ultérieure. De plus, CF₃SCl est un composé gazeux à température ambiante, tandis que CF₃S(O)Cl est un liquide ce qui facilite les manipulations.

Selon un deuxième mode de réalisation particulier de l'invention, on utilise le composé CF₃S(O)X dans lequel X est le groupe N(CH₃)₂ ou N(C₂H₅)₂.

Selon un troisième mode de réalisation particulier de l'invention, on utilise le composé CF₃S(O)X dans lequel X est le groupe hydroxy OH ou ONa et dans ce cas la réaction est faite en présence de phosgène COCl₂ ou SOCl₂.

Le procédé, avec ses modes préférés de réalisation, est particulièrement bien adapté lorsque l'hétérocycle Het est un composé de formule A ou B suivantes 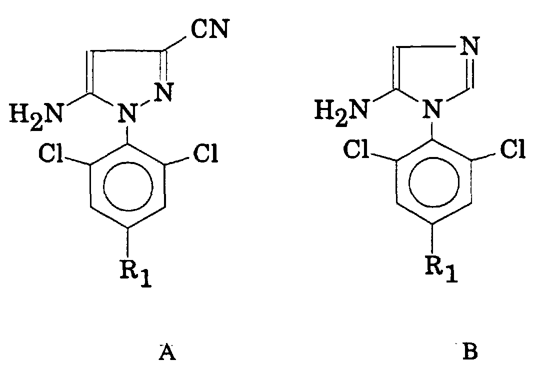 dans lesquelles R₁ représente un atome d'halogène, de préférence le fluor, ou un groupe alkyl ou haloalkyl, de préférence CF₃, ou un groupe SF₅.

L'invention concerne en outre un procédé d'obtention de 4-sulfinylpyrazoles de formules : 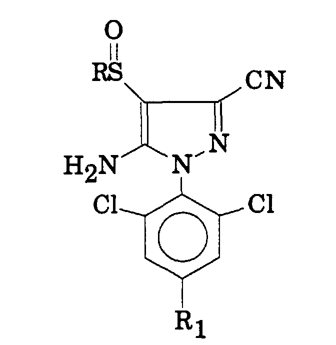 dans lesquelles R est un groupe alkyl ayant de un à quatre atomes de carbone, linéaire ou ramifié, substitué par un ou plusieurs atomes d'halogène identiques ou différents et R₁ représente un atome d'halogène, de préférence le fluor, ou un groupe alkyl ou haloalkyl, de préférence CF₃, ou un groupe SF₅ à partir de composé de formules : 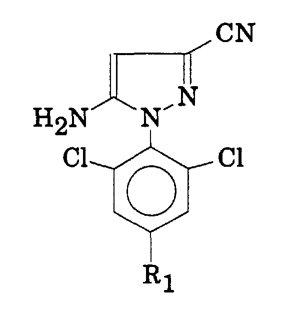 par action d'un réactif de formule RS(O)X, dans laquelle R est un groupe alkyl ayant de un à quatre atomes de carbone, linéaire ou ramifié, substitué par un ou plusieurs atomes d'halogène identiques ou différents et X un atome d'halogène, le groupe hydroxy ou l'un de ses sels, un groupe dialkylamino NR₂R₃, R₂ et R₃ étant des groupes alkyl ou haloakyl de 1 à 4 atomes de carbone, ou un groupe aryloxy, dans lequel la partie aryl correspond de préférence à un groupe phényl, éventuellement substitué par un ou plusieurs atomes d'halogène ou groupes alkyl ou haloalkyl de 1 à 4 atomes de carbone. On opère avec un excès molaire de réactif RS(O)X par rapport au composé 4-H pyrazole ci-dessus. Cet excès est de l'ordre de 10 à 50*%*, de préférence de 20 à 30%.

Pour parfaire la réaction ci-dessus, on utilise avantageusement un composé C choisi dans le groupe comprenant les tosylates, les chlorhydrates, les mésylates de diméthylamine, de pyridine, de triméthylamine, de diéthylamine, d'isopropylamine ou de toute autre amine primaire, secondaire ou tertiaire. Ce composé C peut également être de l'acide chlorhydrique gazeux, éventuellement en présence d'une quantité environ équimolaire d'acide paratoluènesulfonique. Le rapport molaire entre le composé C et le composé hétérocyclique est avantageusement compris entre 0,5 et 2, et de préférence entre 1 et 2. Par ailleurs, la réaction est faite en milieu organique dans un solvant choisi dans le groupe comprenant le toluène, le dichloro-1,2-éthane, le dichlorométhane. La température de réaction est comprise entre 0 et 100°C, de préférence entre 3 et 60°C, de préférence encore entre 30 et 55°C.

Un réactif choisi dans le groupe comprenant le phosgène COCl₂, les chloroformiates, le PCl₅ ou le SOCl₂ peut éventuellement être utilisé pour faire les réactions précitées.

Le procédé de sulfinylation de pyrazoles ci-dessus est encore plus avantageusement utile pour produire les 4-sulfinylpyrazoles de formules :

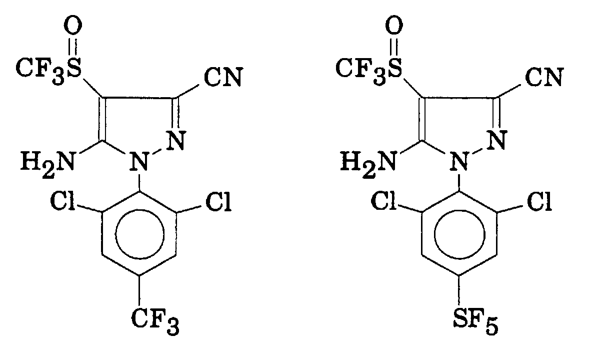

On fait alors réagir soit CF₃S(O)Cl, CF₃S(O)N(CH₃)₂ ou CF₃S(O)N(C₂H₅)₂, soit CF₃S(O)OH ou CF₃S(O)ONa avec du phosgène ou SOCl₂ ou ClCO₂C₂H₅ selon le procédé et ses modes préférentiels décrits, sur un composé 4-H pyrazole ayant une des formules ci-dessous :

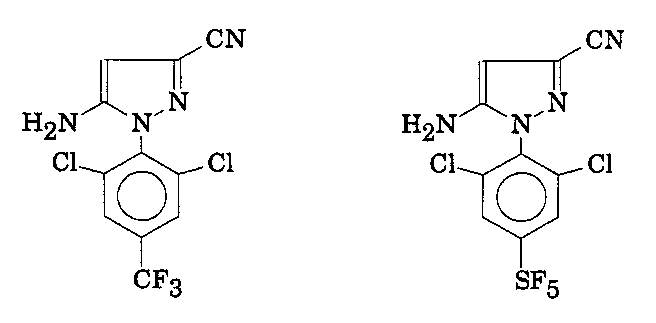

L'invention concerne en outre des composés sulfinamides intermédiaires, obtenus quand le composé hétérocycle porte un groupe NH₂, de formules RS(O)NH-Het, dans lesquels Het est un radical hétérocyclique choisi dans le groupe comprenant les pyrroles, les pyrazoles, les imidazoles, les oxazoles, les isoxazoles, les isothiazoles, les thiazoles, les triazoles, tous ces hétérocycles Het étant éventuellement substitués par un ou plusieurs atomes ou groupes choisi parmi halogène, amine, mono- ou di-alkylamino, nitrile, aryl, aryl substitués par un ou plusieurs atome d'halogène et/ou un ou plusieurs groupes alkyl, haloalkyl ou SF₅.

Plus avantageusement les composés RS(O)NH-Het, où Het est un hétérocycle pyrazole, éventuellement substitué par un ou plusieurs atomes ou groupes choisi parmi halogène, amine, mono- ou di-alkylamino, nitrile, aryl, aryl substitués par un ou plusieurs atome d'halogène et/ou un ou plusieurs groupes alkyl, haloalkyl ou SF₅ et où le groupe sulfinamide est en position 5 sur cet hétérocycle, font partie de la présente invention.

De manière encore plus avantageuse, l'invention concerne les composés 5-(N-trifluorométhylsulfinyl)amino-3-cyano-1-[2,6-dichloro 4-CF3 phényl] 4-H pyrazole et 5-(N-trifluorométhylsulfinyl)amino-3-cyano-1-[2,6-dichloro 4-SF₅ phényl] 4-H pyrazole.

### EXEMPLES :

Les exemples suivants donnés à titre non limitatif illustrent l'invention et montrent comment elle peut être mise en pratique.

### Exemple 1:

### Sulfinylation par CF₃S(O)Cl : synthèse du 5-amino-3-cyano-1-(2,6-dichloro-4-trifluorométhylphényl)-4-trifluorométhylsulfinyl pyrazole.

8,06 g (25 mmoles) de 5-amino-3-cyano-1-(2,6-dichloro-4-trifluorométhylphényl) pyrazole et 8,15 g (38 mmoles) de tosylate de diméthylamine sont placés en suspension dans 50 ml de toluène.

Le chlorure de trifluorométhylsulfinyl (5 g soit 32 mmoles) est rapidement ajouté à ce mélange. Le milieu réactionnel est alors chauffé à 50°C. Après 8 heures de réaction à cette température, la réaction est purgée par un courant d'argon. Le milieu réactionnel est ensuite refroidit à 20°C. On lui ajoute 20 ml d'eau, puis le précipité est filtré, lavé à l'eau puis au toluène.

Le produit obtenu est sèché à chaud sous vide. On obtient ainsi 9,77 g (soit un rendement de 88%) de 5-amino-3-cyano-1-(2,6-dichloro-4-trifluorométhylphényl)-4-trifluorométhylsulfinyl pyrazole, avec une pureté de plus de 95% (déterminée par CLPH).

Les caractéristiques physiques et spectrales du composé obtenu sont les suivantes :
- Point de fusion = 196-198°C.
- Analyse RMN : RMN du proton ¹H, CDCl₃, TMS :
   5,1 ppm (s, 2H), 7,8 ppm (s, 2H).
   RMN du carbone ¹³C, acétone d⁶, TMS :
   groupe phényl : C₁ : 135,4 ppm, C₂ : 137,5 ppm, C₃ : 127,6 ppm, C₄ : 135,5 ppm, C (CF₃):123 ppm.
   groupe pyrazole : C₃ : 126,7 ppm, C₄ : 94,6 ppm, C₅ : 152,3 ppm, C (CN) : 111,7 ppm, C (CF₃) : 126,3 ppm.
- Analyse masse, EI + : M = 436 (35C1).

### Exemple 2 :

### Sulfinylation par CF₃S(O)Cl : synthèse du 5-amino-3-cyano-1-(2,6-dichloro-4-trifluorométhylphényl)-4-trifluorométhylsulfinyl pyrazole.

0,81 g (2,5 mmoles) de 5-amino-3-cyano-1-(2,6-dichloro-4-trifluorométhylphényl) pyrazole et 0,29 g (2,5 mmoles) de chlorhydrate de pyridine sec sont placés en solution dans 5 ml de dichloro-1,2-éthane.

Le chlorure de trifluorométhylsulfinyl (0,5 g soit 3,2 mmoles) est ajouté à ce mélange. Le milieu réactionnel est alors chauffé à 50°C pendant 10 heures. On opère alors comme à l'exemple précédent.

Le rendement dosé en 5-amino-3-cyano-1-(2,6-dichloro-4-trifluorométhylphényl)-4-trifluorométhylsulfinyl pyrazole est alors de 74%.

### Exemple 3 :

### Sulfinylation par CF₃S(O)NMe₂ : synthèse du 5-amino-3-cyano-1-(2,6-dichloro-4-trifluorométhylphényl)-4-trifluorométhylsulfinyl pyrazole.

0,81 g (2,5 mmoles) de 5-amino-3-cyano-1-(2,6-dichloro-4-trifluorométhylphényl) pyrazole et 0,55 g (2,5 mmoles) d'acide paratoluènesulfonique sec sont placés en suspension dans 5 ml de toluène. Le N,N diméthyl trifluorométhylsulfinylamine (0,53 g soit 3,2 mmoles) puis une solution HCl dans du toluène (soit 2,5 mmoles) sont ajoutés dans le milieu. Celui-ci est chauffé 8 heures à 50°C. On opére alors comme dans l'exemple 1.

Le rendement dosé en 5-amino-3-cyano-1-(2,6-dichloro-4-trifluorométhylphényl)-4-trifluorométhylsulfinyl pyrazole est alors de 72%.

### Exemple 4 :

### synthèse du 5-(N-trifluorométhylsulfinyl)amuio-3-cyano-1-(2,6-dichloro-4-trifluorométhylphényl) pyrazole.

Le sulfinamide pyrazole, compris dans la présente invention, peut être préparé et isolé selon les conditions suivantes:

3,23 g (10 mmoles) de 5-amino-3-cyano-1-(2,6-dichloro-4-trifluorométhylphényl) pyrazole et 3,25 g (15 mmoles) de tosylate de diméthylamine sont placés en suspension dans 20 ml de toluène. La température est amenée à 5°C . Le chlorure de trifluororméthylsulfinyl (2g soit 13 mmoles) est ajouté rapidement. On ajoute ensuite une solution toluènique de diméthylamine (5 mmoles).

La réaction est laissée 30 mn sous agitation à 5°C. On ajoute alors 50 ml de méthyl terbutyl éther. Le précipité formé est éliminé par filltration, puis rincé. Le filtrat est récupéré et lavé par extraction par 2x10 ml d'eau glacée. La phase organique est concentrée. Le résidu obtenu est cristallisé dans le toluène.

On obtient ainsi 1,75 g de 5-(N-trifluorométhylsulfinyl)amino-3-cyano-1-(2,6-dichloro-4-trifluorométhylphényl) pyrazole.

Les caractéristiques physiques et spectrales du composé obtenu sont les suivantes :
- Point de fusion = 123-124°C.
- Analyse RMN : RMN du proton ¹H, acétone d⁶, HMDS :
   7,06 ppm (s, 1p), 8,06 ppm (s, 2p).
   RMN du carbone ¹³C, acétone d⁶, TMS :
   groupe phényl : C₁ : 136,2 ppm, C₂ : 137 ppm, C₃ : 127,4 ppm, C₄ : 135,2 ppm, C (CF₃) : 123,3 ppm.
   groupe pyrazole : C₃ : 128,5 ppm, C₄ : 105,7 ppm, C₅ : 140,7 ppm, C (CN) : 113,5 ppm, C (CF₃) : 124,5 ppm.

### Exemple 5 :

### Synthèse du 5-amino-3-cyano-1-(2,6-dichloro-4-trifluorométhylphényl)-4 phényl)-4-trifluorométhylsulfinyl pyrazole par réarrangement du sulfinamide pyrazole de l'exemple 4.

0,109 g (0,25 mmoles) de sulfinamide pyrazole, obtenu à l'exemple précédent, et 0,075 g (0,33 mmoles) de tosylate de diméthylamine sont placés en suspension du toluène. On ajoute une solution d'acide chlorhydrique dans du toluène (soit 0,25 mmoles d'HCl). Le milieu réactionnel est chauffé 10 heures à 50°C.

Le rendement dosé en 5-amino-3-cyano-1-(2,6-dichloro-4-trifluorométhyl phényl)-4-trifluorométhylsulfinyl pyrazole est de 80%.

### Exemple 6 :

### Sulfinylation par CF₃S(O)ONa : synthèse du 5-amino-3-cyano-1-(2,6-dichloro-4-trifluorométhylphényl)-4-trifluorométhylsulfinyl pyrazole.

0,81 g (2,5 mmoles) de 5-amino-3-cyano-1-(2,6-dichloro-4-trifluorométhylphényl) pyrazole, 0,815 g (3,8 mmoles) de tosylate de diméthylamine et 0,51 g (3,25 mmoles) de sel de sodium de l'acide trifluorométhanesulfinique sont placés en suspension dans 5 ml de toluène. A ce mélange refroidi à 5°C, on ajoute SOCl₂ puis on laisse sous agitation environ 1 heure à température ambiante. Le milieu réactionnel est ensuite chauffé à 50°C pendant 8 heures. On opére alors comme dans l'exemple 1.

Le rendement dosé en 5-amino-3-cyano-1-(2,6-dichloro-4-trifluorométhyl phényl)-4-trifluorométhylsulfinyl pyrazole est de 66%.

## Revendications

1. Procédé de sulfinylation de composés hétérocycliques **caractérisé en ce que** l'on fait réagir un dérivé de formule RS(O)X, dans laquelle R est un groupe halométhyle, X est un atome d'halogène, le groupe hydroxy ou l'un de ses sels, un groupe dialkylamino NR₂R₃, R₂ et R₃ étant des groupes alkyl ou haloalkyl de 1 à 4 atomes de carbone, ou un groupe aryloxy, dans lequel la partie aryl correspond de préférence à un groupe phényl, éventuellement substitué par un ou plusieurs atomes d'halogène ou groupes alkyl ou haloalkyl de 1 à 4 atomes de carbone, sur un composé hétérocyclique Het choisi dans le groupe comprenant les pyrroles, les pyrazoles, les imidazoles, les oxazoles, les isoxazoles, les isothiazoles, les thiazoles, les triazoles, tous ces hétérocycles Het étant éventuellement substitués par un ou plusieurs atomes ou groupes choisi parmi halogène, amine, mono- ou di-alkylamino, nitrile, aryl, aryl substitués par un ou plusieurs atome d'halogène et/ou un ou plusieurs groupes alkyl, haloalkyl ou SF₅.

2. Procédé selon la revendication 1 **caractérisé en ce que** Het est choisi dans le groupe comprenant les pyrroles, les pyrazoles, les imidazoles et **en ce qu'**un composé choisi dans le groupe comprenant les tosylates, les chlorhydrates, les mésylates de diméthylamine, de pyridine, de triméthylamine, de diéthylamine, d'isopropylamine, ou de toute autre amine primaire, secondaire ou tertiaire, ou l'acide chlorhydrique, éventuellement en présence d'acide paratoluènesulfonique, est utilisé pour faire la réaction.

3. Procédé selon l'une des revendications 1 ou 2 **caractérisé en ce qu'**un réactif choisi dans le groupe comprenant le phosgène COCl₂, les chloroformiates, le PCl₅ ou le SOCl₂ est utilisé pour faire la réaction.

4. Procédé selon l'une des revendications 1 à 3 **caractérisé en ce que** Het est un groupe aminopyrazole qui réagit avec RS(O)X pour donner un composé sulfinamide se réarrangeant par la suite pour conduire à un composé hétérocyclique ayant un groupe amino porté par un atome de carbone et un groupe sulfinyl RS(O) sur le carbone vicinal.

5. Procédé selon l'une des revendications 1 à 4 **caractérisé en ce que** R est le groupe trifluorométhyl CF₃ et X l'atome de chlore.

6. Procédé selon l'une des revendications 1 à 4 **caractérisé en ce que** R est le groupe trifluorométhyl CF₃ et X le groupe N(CH₃)₂ ou N(C₂H₅)₂.

7. Procédé selon l'une des revendications 3 ou 4 **caractérisé en ce que** R est le groupe trifluorométhyl CF₃ et X le groupe hydroxy OH ou ONa et **en ce que** la réaction est faite en présence de phosgène COCl₂ ou SOCl₂.

8. Procédé selon l'une des revendications 1 à 7 **caractérisé en ce que** l'hétérocycle Het est un composé de formule A ou B suivantes : 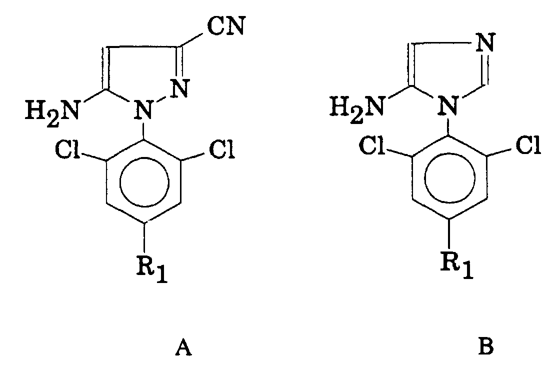 dans lesquelles R₁ représente un atome d'halogène, de préférence le fluor, ou un groupe alkyl ou haloalkyl, de préférence CF₃, ou un groupe SF₅.

9. Procédé d'obtention de 4-sulfinylpyrazoles de formules : 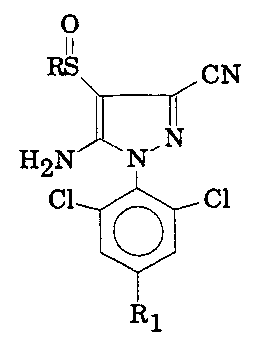 dans lesquelles R est un groupe alkyl ayant de un à quatre atomes de carbone, linéaire ou ramifié, substitué par un ou plusieurs atomes d'halogène identiques ou différents et R₁ représente un atome d'halogène, de préférence le fluor, ou un groupe alkyl ou haloalkyl, de préférence CF₃, ou un groupe SF₅ **caractérisé en ce que** l'on fait réagir sur un composé de formules : 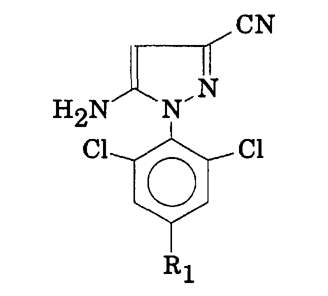 un réactif de formule RS(O)X, dans laquelle R est un groupe alkyl ayant de un à quatre atomes de carbone, linéaire ou ramifié, substitué par un ou plusieurs atomes d'halogène identiques ou différents et X un atome d'halogène, le groupe hydroxy ou l'un de ses sels, un groupe diakylamino NR₂R₃, R₂ et R₃ étant des groupes alkyl ou haloalkyl de 1 à 4 atomes de carbone, ou un groupe aryloxy, dans lequel la partie aryl correspond de préférence à un groupe phényl, éventuellement substitué par un ou plusieurs atomes d'halogène ou groupes alkyl ou haloalkyl de 1 à 4 atomes de carbone.

10. Procédé selon la revendication 9 **caractérisé en ce qu'**un composé choisi dans le groupe comprenant les tosylates, les chlorhydrates, les mésylates de diméthylamine, de pyridine, de triméthylamine, de diéthylamine, d'isopropylamine ou de toute autre amine primaire, secondaire ou tertiaire, ou l'acide chlorhydrique éventuellement en présence d'acide paratoluènesulfonique, est utilisé pour faire la réaction.

11. Procédé selon l'une des revendications 9 ou 10 **caractérisé en ce qu'**un réactif choisi dans le groupe comprenant le phosgène COCl₂, les chloroformiates, le PCl₅ ou le SOCl₂ est utilisé pour faire la réaction.

12. Procédé selon l'une des revendications 9 à 11 d'obtention de 4-sulfinylpyrazoles de formules : 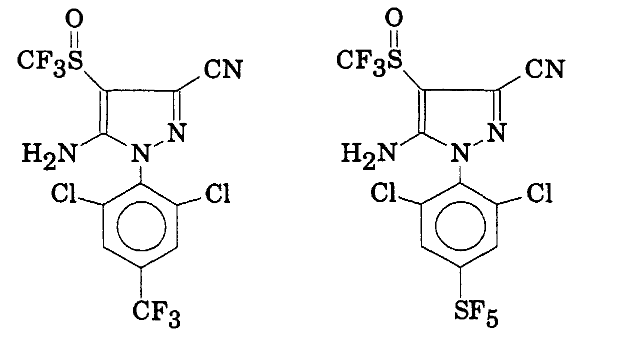 par action :
- soit de CF₃S(O)Cl, CF₃S(O)N(CH₃)₂ ou CF₃S(O)N(C₂H₅)₂,
- soit de CF₃S(O)OH ou CF₃S(O)ONa avec du phosgène ou SOCl₂ ou ClCO₂C₂H₅
sur un composé 4-H pyrazole ayant une des formules ci-dessous:

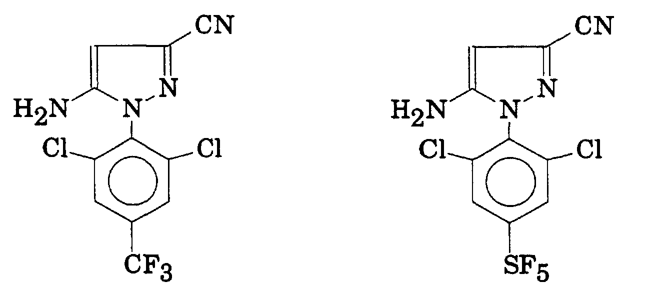

13. Composés sulfinamides intermédiaires de formules RS(O)NH-Het, dans lesquelles Het a la définition donnée à la revendication 1 ou 2.

14. Composés selon la revendication 13 **caractérisé en ce que** Het est un hétérocycle pyrazole, éventuellement substitué par un ou plusieurs atomes ou groupes choisi parmi halogène, amine, mono- ou di-alkylamino, nitrile, aryl, aryl substitués par un ou plusieurs atome d'halogène et/ou un ou plusieurs groupes alkyl, haloalkyl ou SF₅ et **en ce que** le groupe sulfinamide est en position 5 sur cet hétérocycle.

15. Composé selon la revendication 14 **caractérisé en ce que** R est le groupe trifluorométhyl CF₃ et Het est 1-[2,6-dichloro 4-CF₃ phényl] 3-cyano 4-H pyrazole.

16. Composé selon la revendication 14 **caractérisé en ce que** R est le groupe trifluorométhyl CF₃ et Het est 1-[2,6-dichloro 4-SF₅ phényl] 3-cyano 4-H pyrazole.

## Patentansprüche

1. Verfahren zur Sulfinylierung von heterocyclischen Verbindungen, **dadurch gekennzeichnet, daß** ein Derivat der Formel RS(O)X, worin R Halomethyl bedeutet und X ein Halogenatom, die Hydroxygruppe oder eines ihrer Salze, eine Dialkylaminogruppe NR₂R₃, wobei R₂ und R₃ Alkyl- oder Haloalkylgruppen mit 1 bis 4 Kohlenstoffatomen sind, oder eine Aryloxygruppe ist, wobei die Arylgruppe vorzugsweise eine Phenylgruppe ist, die gegebenenfalls mit einem oder mehreren Halogenatomen oder einer oder mehreren Alkyl- oder Haloalkylgruppen mit 1 bis 4 Kohlenstoffatomen substituiert ist, mit einer heterocyclischen Verbindung Het umgesetzt wird, die unter den Pyrrolen, Pyrazolen, Imidazolen, Oxazolen, Isoxazolen, Isothiazolen, Thiazolen, Triazolen oder den vorgenannten Heterocyclen Het ausgewählt ist, die gegebenenfalls mit einem oder mehreren Atomen oder einer oder mehreren Gruppen substituiert sind, die unter Halogen, Amin, Mono- oder Dialkylamino, Nitril, Aryl und Arylgruppen ausgewählt sind, die mit einem oder mehreren Halogenatomen und/oder einer oder mehreren Gruppen Alkyl, Haloalkyl oder SF₅ substituiert sind.

2. Verfahren nach Anspruch 1, **dadurch gekennzeichnet, daß** Het unter den Pyrrolen, Pyrazolen und Imidazolen ausgewählt ist und dadurch, daß eine Verbindung, die unter den Tosylaten, Hydrochloriden und Mesylaten von Dimethylamin, Pyridin, Trimethylamin, Diethylamin Isopropylamin oder beliebigen weiteren primären, sekundären oder tertiären Aminen oder Salzsäure gegebenenfalls in Gegenwart von p-Toluolsulfonsäure ausgewählt ist, zur Durchführung der Umsetzung verwendet wird.

3. Verfahren nach einem der Ansprüche 1 oder 2, **dadurch gekennzeichnet, daß** ein Reaktant, der unter Phosgen COCl₂, Chlorformiaten, PCl₅ oder SOCl₂ ausgewählt ist, zur Durchführung der Umsetzung verwendet wird.

4. Verfahren nach einem der Ansprüche 1 bis 3, **dadurch gekennzeichnet, daß** Het Aminopyrazol bedeutet, das mit RS(O)X unter Bildung eines Sulfinamids reagiert, das sich in der Folge umlagert, wodurch eine heterocyclische Verbindung gebildet wird, die eine Aminogruppe an einem Kohlenstoffatom und eine Sulfinylgruppe RS(O) an dem benachbarten Kohlenstoffatom aufweist.

5. Verfahren nach einem der Ansprüche 1 bis 4, **dadurch gekennzeichnet, daß** R die Trifluormethylgruppe CF₃ ist und X Chlor bedeutet.

6. Verfahren nach einem der Ansprüche 1 bis 4, **dadurch gekennzeichnet, daß** R die Trifluormethylgruppe CF₃ ist und X N(CH₃)₂ oder N(C₂H₅)₂ bedeutet.

7. Verfahren nach einem der Ansprüche 3 oder 4, **dadurch gekennzeichnet, daß** R die Trifluormethylgruppe CF₃ ist und X Hydroxy OH oder. ONa bedeutet und dadurch, daß die Umsetzung in Gegenwart von Phosgen COCl₂ oder SOCl₂ durchgeführt wird,

8. Verfahren nach einem der Ansprüche 1 bis 7, **dadurch gekennzeichnet, daß** der Heterocyclus Het eine Verbindung der folgenden Formel A oder B ist: 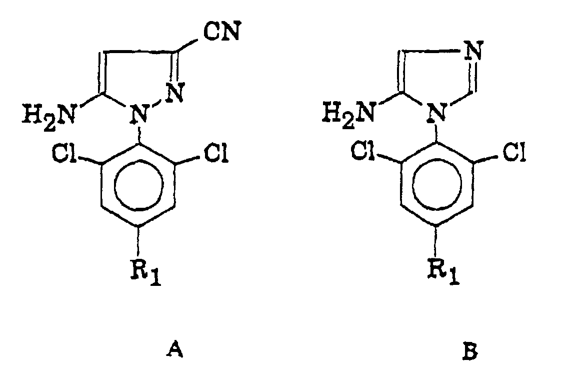 worin R₁ ein Halogenatom und vorzugsweise Fluor oder eine Alkyl- oder Haloalkylgruppe, vorzugsweise CF₃, oder eine Gruppe SF₅ bedeutet.

9. Verfahren zur Herstellung von 4-Sulfinylpyrazolen der Formel: 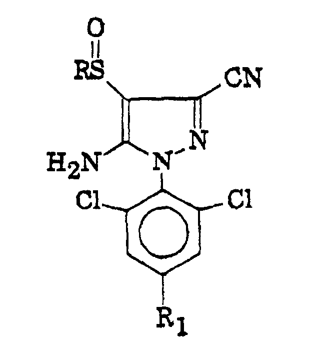 worin R eine geradkettige oder verzweigte Alkylgruppe mit 1 bis 4 Kohlenstoffatomen ist, die mit einem oder mehreren Halogenatomen substituiert ist, welche identisch oder voneinander verschieden sind, und R₁ ein Halogenatom und vorzugsweise Fluor, eine Alkyl- oder Haloalkylgruppe, vorzugsweise CF₃, oder die Gruppe SF₅ bedeutet, **dadurch gekennzeichnet, daß** mit einer Verbindung der Formel: 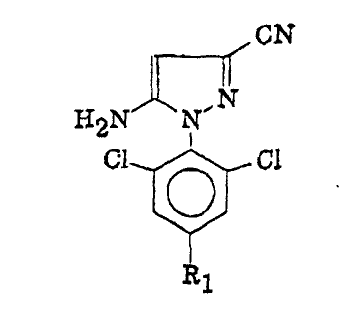 ein Reaktant der Formel RS(O)X umgesetzt wird, wobei R eine geradkettige oder verzweigte Alkylgruppe mit 1 bis 4 Kohlenstoffatomen ist, die mit einem oder mehreren Halogenatomen, die identisch oder voneinander verschieden sind, substituiert ist, und X ein Halogenatom, die Hydroxygruppe oder eines ihrer Salze, eine Gruppe Dialkylamino NR₂R₃, wobei R₂ und R₃ Alkyloder Haloalkylgruppen mit 1 bis 4 Kohlenstoffatomen bedeuten, oder eine Aryloxygruppe ist, wobei die Arylgruppe vorzugsweise Phenyl bedeutet, die gegebenenfalls mit einem oder mehreren Halogenatomen oder einer oder mehreren Alkyl- oder Haloalkylgruppen mit 1 bis 4 Kohlenstoffatomen substituiert ist.

10. Verfahren nach Anspruch 9, **dadurch gekennzeichnet, daß** eine Verbindung, die unter den Tosylaten, Hydrochloriden und Mesylaten von Dimethylamin, Pyridin, Trimethylamin, Diethylamin, Isopropylamin oder von beliebigen weiteren primären, sekundären oder tertiären Aminen oder Salzsäure gegebenenfalls in Gegenwart von p-Toluolsulfonsäure ausgewählt ist, zur Durchführung der Umsetzung verwendet wird.

11. Verfahren nach einem der Ansprüche 9 oder 10, **dadurch gekennzeichnet, daß** ein Reaktant, der unter Phosgen COCl₂, Chlorformiaten, PCl₅ oder SOCl₂ ausgewählt ist, zur Durchführung der Umsetzung verwendet wird.

12. Verfahren nach einem der Ansprüche 9 bis 11 zur Herstellung von 4-Sulfinylpyrazolen der Formeln: 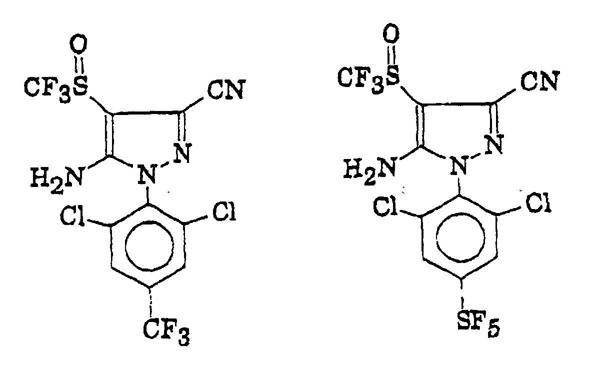 durch Umsetzung von:
- entweder CF₃S(O)Cl, CF₃S(O)N(CH₃)₂ oder CF₃S(O)N(C₂H₅)₂,
- oder CF₃S(O)OH oder CF₃S(O)ONa mit Phosgen oder SOCI₂ oder ClCO₂C₂H₅
mit einem 4-H-Pyrazol, das eine der folgenden Formeln aufweist:

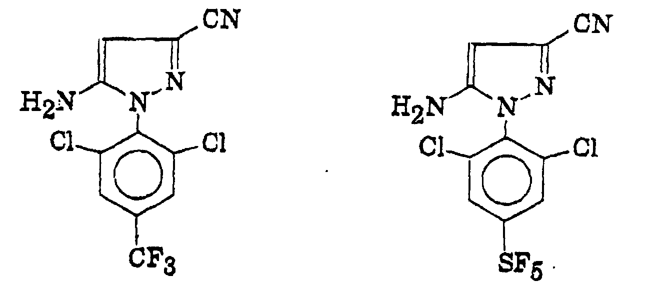

13. Sulfinamid-Zwischenprodukte der Formel RS(O)NH-Het, worin Het die in Anspruch 1 oder 2 angegebenen Bedeutungen aufweist.

14. Verbindungen nach Anspruch 13, **dadurch gekennzeichnet, daß** Het ein Pyrazol-Heterocyclus ist, der gegebenenfalls mit einem oder mehreren Atomen oder einer oder mehreren Gruppen substituiert ist, die unter Halogen, Amin, Mono- oder Dialkylamino, Nitril, Aryl und Arylgruppen ausgewählt sind, die mit einem oder mehreren Halogenatomen und/oder einer oder mehreren Gruppen Alkyl, Haloalkyl oder SF₅ substituiert sind, und dadurch, daß sich die Sulfinamidgruppe am Heterocyclus in 5-Stellung befindet.

15. Verbindungen nach Anspruch 14, **dadurch gekennzeichnet, daß** R die Trifluormethylgruppe CF₃ ist und Het 1-[2,6-Dichlor-4-CF₃-phenyl]-3-cyano-4-H-pyrazol bedeutet.

16. Verbindung nach Anspruch 14, **dadurch gekennzeichnet, daß** R die Trifluormethylgruppe CF₃ ist und Het 1-[2,6-Dichlor-4-SF₅phenyl]-3-cyano-4-H-pyrazol bedeutet.

## Claims

1. Process for the sulphinylation of heterocyclic compounds, **characterized in that** a derivative of formula RS(O)X, in which R is a halomethyl group, X is a halogen atom, the hydroxyl group or one of the salts thereof, a dialkylamino group NR₂R₃, R₂ and R₃ being alkyl or haloalkyl groups of 1 to 4 carbon atoms, or an aryloxy group, in which the aryl part preferably corresponds to a phenyl group, which is optionally substituted with one or more halogen atoms or alkyl or haloalkyl groups of 1 to 4 carbon atoms, is reacted with a heterocyclic compound Het chosen from the group comprising pyrroles, pyrazoles, imidazoles, oxazoles, isoxazoles, isothiazoles, thiazoles or triazoles, all of these heterocycles Het optionally being substituted with one or more atoms or groups chosen from halogen, amine, mono-or dialkylamino, nitrile, aryl, aryl substituted with one or more halogen atoms and/or one or more alkyl, haloalkyl or SF₅ groups.

2. Process according to Claim 1, **characterized in that** Het is chosen from the group comprising pyrroles, pyrazoles and imidazoles and **in that** a compound chosen from the group comprising the tosylates, hydrochlorides and mesylates of dimethylamine, of pyridine, of trimethylamine, of diethylamine, of isopropylamine or of any other primary, secondary or tertiary amine, or hydrochloric acid, optionally in the presence of para-toluenesulphonic acid, is used to perform the reaction.

3. Process according to either of Claims 1 and 2, **characterized in that** a reactant chosen from the group comprising phosgene COCl₂, chloroformates, PCl₅ or SOCl₂ is used to perform the reaction.

4. Process according to one of Claims 1 to 3, **characterized in that** Het is an aminopyrazole group which reacts with RS(O)X to give a sulphinamide compound which subsequently rearranges to give a heterocyclic compound having an amino group borne by one carbon atom and a sulphinyl group RS(O) on the vicinal carbon.

5. Process according to one of Claims 1 to 4, **characterized in that** R is the trifluoromethyl group CF₃ and X is a chlorine atom.

6. Process according to one of Claims 1 to 4, **characterized in that** R is the trifluoromethyl group CF₃ and X is the N(CH₃)₂ or N(C₂H₅)₂ group.

7. Process according to either of Claims 3 and 4 **characterized in that** R is the trifluoromethyl CF₃ and X is the hydroxyl OH or ONa group and **in that** the reaction is performed in the presence of phosgene COCl₂ or SOCl₂.

8. Process according to one of Claims 1 to 7, **characterized in that** the heterocycle Het is a compound of formula A or B below: 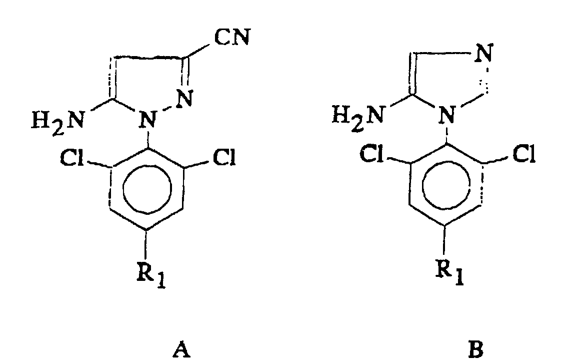 in which R₁ represents a halogen atom, preferably fluorine, or an alkyl or haloalkyl group, preferably CF₃, or an SF₅ group.

9. Process for the production of 4-sulphinylpyrazoles of formula: 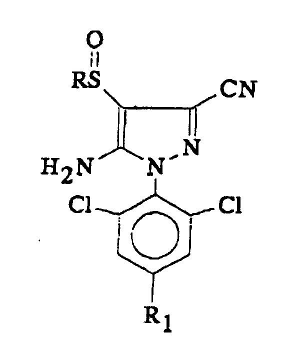 in which R is a linear or branched alkyl group having from one to four carbon atoms, which is substituted with one or more identical or different halogen atoms and R₁ represents a halogen atom, preferably fluorine, or an alkyl or haloalkyl group, preferably CF₃, or an SF₅ group, **characterized in that** a compound of formula: 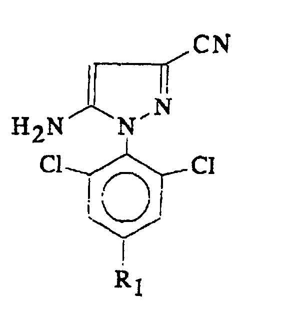 is reacted with a reactant of formula RS(O)X, in which R is a linear or branched alkyl group having from one to four carbon atoms, which is substituted with one or more identical or different halogen atoms and X is a halogen atom, the hydroxyl group or one of the salts thereof, a dialkylamino group NR₂R₃, R₂ and R₃ being alkyl or haloalkyl groups of 1 to 4 carbon atoms, or an aryloxy group, in which the aryl part preferably corresponds to a phenyl group, which is optionally substituted with one or more halogen atoms or alkyl or haloalkyl groups of 1 to 4 carbon atoms.

10. Process according to Claim 9, **characterized in that** a compound chosen from the group comprising the tosylates, hydrochlorides and mesylates of dimethylamine, of pyridine, of trimethylamine, of diethylamine, of isopropylamine or of any other primary, secondary or tertiary amine, or hydrochloric acid optionally in the presence of para-toluenesulphonic acid, is used to perform the reaction.

11. Process according to either of Claims 9 and 10, **characterized in that** a reactant chosen from the group comprising phosgene COCl₂, chloroformates, PCl₅ or SOCl₂ is used to perform the reaction.

12. Process according to one of Claims 9 to 11 for the production of 4-sulphinylpyrazoles of formulae: 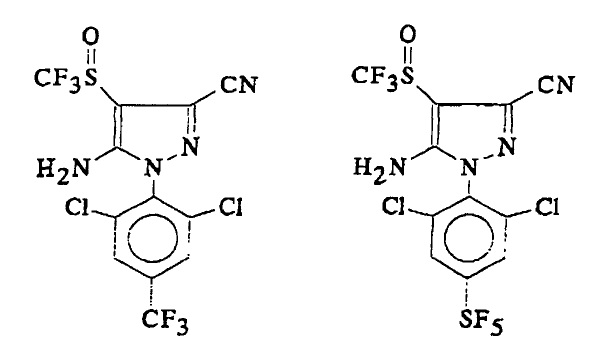 by the action:
- either of CF₃S(O)Cl, CF₃S (O)N(CH₃)_{2 2} or CF₃S(O)N(C₂H₅)₂,
- or of CF₃S(O)OH or CF₃S(O)ONa with phosgene or SOCl₂ or ClCO₂C₂H₅
on a 4-H pyrazole compound having one of the formulae below:

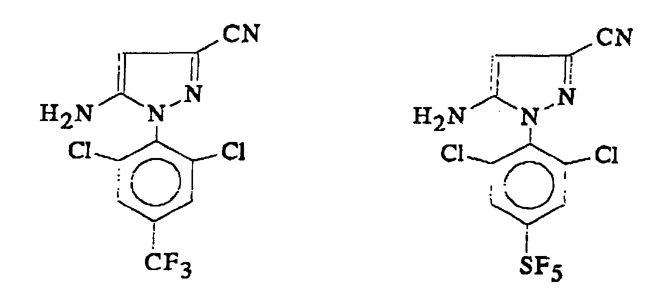

13. Intermediate sulphinamide compounds of formula RS(O)NH-Het, in which Het has the definition given in Claim 1 or 2.

14. Compounds according to Claim 13, **characterized in that** Het is a pyrazole heterocycle, which is optionally substituted with one or more atoms or groups chosen from halogen, amine, mono- or dialkylamino, nitrile, aryl, aryl substituted with one or more halogen atoms and/or one or more alkyl, haloalkyl or SF₅ groups and **in that** the sulphinamide group is in position 5 on this heterocycle.

15. Compound according to Claim 14, **characterized in that** R is the trifluoromethyl group CF₃ and Het is 1-[2,6-dichloro-4-CF₃-phenyl]-3-cyano-4-H-pyrazole.

16. Compound according to Claim 14, **characterized in that** R is the trifluoromethyl group CF₃ and Het is 1-[2,6-dichloro-4-SF₅-phenyl]-3-cyano-4-H-pyrazole.
